# EUROPEAN PATENT APPLICATION

(11) **EP 2 199 793 A1**
(43) Date of publication of application: **23.06.2010**
(21) Application number: 08830949.7
(22) Date of filing: 09.09.2008
(51) Int. Cl.: G01N 33/50, A61K 35/12, A61P 35/00, A61P 37/02, C12N 5/10, C12Q 1/02, G01N 33/15, G01N 33/53

(54) **METHOD OF EVALUATING HUMAN DENDRITIC CELLS AND HUMAN CELL IMMUNOTHERAPEUTIC AGENT**

(30) Priority: 10.09.2007 JP 2007234734
(71) Applicant: Riken, Wako-shi, Saitama 351-0198 (JP)
(72) Inventor: FUJII, Shin-ichiro, Yokohama-shi Kanagawa 230-0045 (JP); SHIMIZU, Kanako, Yokohama-shi Kanagawa 230-0045 (JP)
(74) Representative: Duckworth, Timothy John
(86) International application number: PCT/JP2008/066210
(87) International publication number: WO 2009/034961

(57) **Abstract**

Provided is a method of evaluating the antigen presentation potential of human dendritic cells, including the following steps:
(1) a step for administering α-galactosylceramide-pulsed human dendritic cells to a non-human mammal;
(2) a step for collecting a sample containing NKT cells from the non-human mammal;
(3) a step for detecting the activation of NKT cells present in the sample.

Further provided is an agent for human NKT cell immunotherapy, containing human dendritic cells that have been assessed by the method as those possessing an antigen presentation potential for NKT cells.

## Description

### Technical Field

The present invention relates to an assay method enabling an evaluation of the efficacy of human dendritic cells used in human NKT cell immunotherapy, an agent for human NKT cell immunotherapy, and the like.

### Background Art

It has been suggested that NKT cells may mediate the regulatory control of immune reactions as self-reactive T cells, and also be involved in the immunosurveillance of cancer cells to have an anti-tumor effect. NKT cells are known to recognize a class I-like molecule called CD1d, which is not linked to MHC, and to react to glycolipid ligands. Therefore, when stimulated specifically, NKT cells are expected to attack cancer cells without dependence on MHC. In fact, it has been reported that human Vα24⁺ NKT cells, once activated, have an anti-tumor effect on a wide variety of cancers without dependence on MHC (Non-patent Document 1).

α-Galactosylceramides are synthetic glycolipid ligands that bind to CD1d molecules on dendritic cell surfaces to activate NKT cells specifically. Supplies of α-galactosylceramides meeting a drug reference value for human administration (GMP grade) are available. Hence, in recent years, attempts of cancer immunotherapy have been made to treat cancers by administering α-galactosylceramide-pulsed dendritic cells to cancer patients via veins to activate their NKT cells. Regarding this cancer immunotherapy, clinical studies in cancer patients (corresponding to Phase I) have already been completed, and the safety of the therapeutic method has been confirmed. However, because the dendritic cells are isolated from the cancer-bearing patient himself or herself, the dendritic cells used in the immunotherapy as they can have abnormalities, and the pulse conditions sufficient to load α-galactosylceramides cannot always be common among different supplies of dendritic cells. At present, however, there is no established system for determining whether or not α-galactosylceramide-pulsed human dendritic cells are effective as antigen-presenting cells for human NKT cells. Therefore, there are some cases where it is impossible to determine whether a problem exists in the antigen presentation capability of the dendritic cells, or another patient factor is the cause, if interferon-γ is not produced from NKT cells when the human NKT cell immunotherapy is performed.

As a method of evaluating the efficacy of human dendritic cells, a method using a human NKT cell line is possible. A human NKT cell line can be established by culturing human primary NKT cells in the presence of α-galactosylceramide and interleukin-2 (Non-patent Document 2). However, established human NKT cell lines exhibit much higher affinity for the ligand than do human primary NKT cells, so the cell lines are thought to differ in properties from the human NKT cells existing in peripheral blood. Therefore, the results obtained using a human NKT cell line are unlikely to reflect the in vivo activation function purely. Furthermore, human NKT cell lines are not stable for long times. For these reasons, it is not always appropriate to use a human NKT cell line to evaluate the antigen presentation potential of human dendritic cells.

A method using NKT cells of the very patient in need of treatment is also possible to evaluate the antigen presentation potential of dendritic cells. However, first, NKT cells occur only in extremely small amounts in the blood, usually at 0.1% or less of peripheral blood lymphocytes, so a large amount of peripheral blood is required if a number of NKT cells sufficient for the evaluation is to be obtained. Second, in cancer patients who are subjects of NKT cell immunotherapy, decreased numbers of NKT cells and functional defects are observed (see, for example, Non-patent Document 3); therefore, if no antigen presentation potential is observed, it cannot be determined whether dendritic cells are responsible for the cause, or a functional defect in the patient's NKT cells is the cause. For these two reasons, it is unrealistic to use the patient's own NKT cells for evaluation of the efficacy of dendritic cells.

It has already been reported that the CD1d molecule is highly conserved among mammals (Non-patent Document 4), and that mouse NKT cells recognize α-galactosylceramide-pulsed human dendritic cells (Non-patent Documents 1 and 3). However, it has not been reported to date that administration of human dendritic cells to individual mice actually produced immune responses.
As the situation stands, there is no effective means enabling a previous evaluation of the quality of human dendritic cells for use in human NKT cell immunotherapy. Non-patent Document 1: Cancer Res.59, 5102-5105, 1999 Non-patent Document 2: J. Immunol. Meth. 272, 147-159, 2003 Non-patent Document 3: J. Immunol. 177, 3484-3492, 2006 Non-patent Document 4: Immunogenetics 50, 146-151, 1999

### Disclosure of the Invention

### Problems to Be Solved by the Invention

Accordingly, the present invention is directed to providing a new method of quantitatively evaluating the efficacy of the antigen presentation potential of human dendritic cells used in human NKT cell immunotherapy, and to providing an agent for human NKT cell immunotherapy and the like.

### Means for Solving the Problems

The present inventors conducted extensive investigations with the aim of accomplishing the above-described objects, and found that when α-galactosylceramide-pulsed human dendritic cells are administered to mice, mouse NKT cells are unexpectedly activated. Additionally, it was shown that this activation of mouse NKT cells by human dendritic cells correlates with the activation of mouse NKT cells by mouse dendritic cells, and has a quantitative nature. Using this method, it is possible to determine whether or not the dendritic cells to be used in NKT cell immunotherapy are dendritic cells capable of activating NKT cells, and to sort the dendritic cells before being administered to humans. Based on these results, the present inventors have completed the present invention.

Accordingly, the present invention relates to the following.
(1) A method of evaluating the antigen presentation potential of human dendritic cells, comprising the following steps:
   (a) a step for administering α-galactosylceramide-pulsed human dendritic cells to a non-human mammal;
   (b) a step for collecting a sample containing NKT cells from the non-human mammal;
   (c) a step for detecting the activation of NKT cells present in the sample.
(2) The method of the above-described (1), wherein the non-human mammal is a mouse.
(3) The method of the above-described (1) or (2), wherein the sample containing NKT cells is derived from a spleen, liver or peripheral blood.
(4) The method of any one of the above-described (1) to (3), wherein the activation of NKT cells is evaluated with one or more kinds of cytokines produced by the NKT cells as an index.
(5) The method of the above-described (4), wherein the cytokines include interferon-γ and/or interleukin-4.
(6) The method of any one of the above-described (1) to (5), wherein the method is for sorting out human dendritic cells to be used in human NKT cell immunotherapy.
(7) The method of the above-described (6), wherein the human dendritic cells are derived from a patient with a disease for which a therapeutic effect can be obtained by NKT cell immunotherapy.
(8) The method of the above-described (7), wherein the disease is a cancer.
(9) An agent for human NKT cell immunotherapy, comprising human dendritic cells that have been assessed by the method of the above-described (6) or (7) as those possessing an antigen presentation potential for NKT cells.
(10) The agent of the above-described (9), wherein the agent is for treatment of a cancer.
(11) A method of screening for a ligand capable of activating human NKT cells, comprising the following steps:
   (a) a step for administering human dendritic cells pulsed with a test substance to a non-human mammal;
   (b) a step for collecting a sample containing NKT cells from the non-human mammal;
   (c) a step for detecting the activation of NKT cells present in the sample.
(12) The method described in (10) above, further comprising performing the foregoing steps (a) to (c) using an α-galactosylceramide in place of the test substance, and comparing the degree of activation of NKT cells between a case where the cells are pulsed with the test substance and a case where the cells are pulsed with an α-galactosylceramide.
(13) The method described in (11) or (12) above, wherein the non-human mammal is a mouse.

### Effect of the Invention

Using the method of the present invention, it is possible to sort human dendritic cells to be used in human NKT cell immunotherapy, and it is also possible to evaluate the degree of the antigen presentation capability. The method of the present invention can also be used for efficacy monitoring in patients undergoing human NKT cell immunotherapy. Furthermore, the method of the present invention is also useful as a method of screening for a novel ligand. The agent of the present invention is useful in efficiently causing cancer regression.

### Brief Description of the Drawings

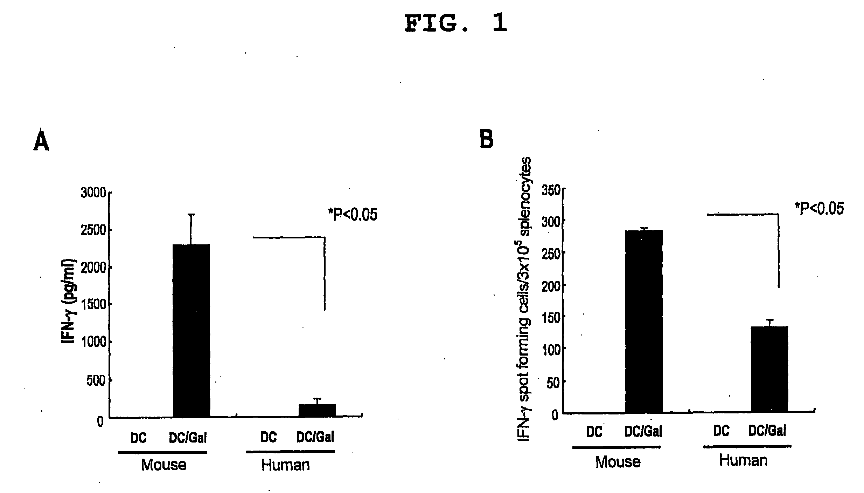

[Figure 1] A drawing showing the activation of mouse NKT cells by administration of hDC/Gal in vitro and in vivo. A. 1×10⁵ cells of mouse DC (mDC) or mDC/Gal, or human DC (hDC) or hDC/Gal, were cultured along with 1×10⁵ cells of C57BL/6 mouse liver mononuclear cells. B. 1×10⁶ cells of mDC or mDC/Gal, and hDC or hDC/Gal, were administered to C57BL/6 mice. Two days later, splenocytes of the immunized mice were measured by IFN-γ ELISPOT assay. The data shown are means for values obtained in three independent experiments. (^{*}) indicates p<0.05.

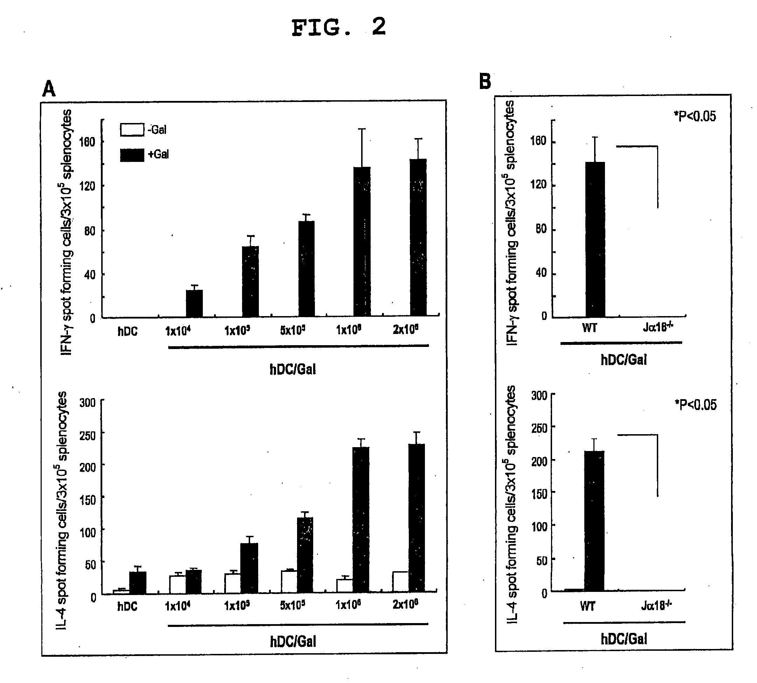

[Figure 2] A drawing showing the activation of mouse NKT cells after administration of hDC/Gal. hDC/Gal was administered to C57BL/6 mice at stepwise doses (A). Alternatively, 1×10⁶ cells of hDC/Gal were administered to Jα18^{-/-} mice (B). Two days later, splenocytes of the immunized mice were cultured in the presence or absence of αGC for 16 hours, and then measured by IFN-γ and IL-4 ELISPOT assay. The data are representative ones for at least two independent experiments yielding similar results. (^{*}) indicates p<0.05.

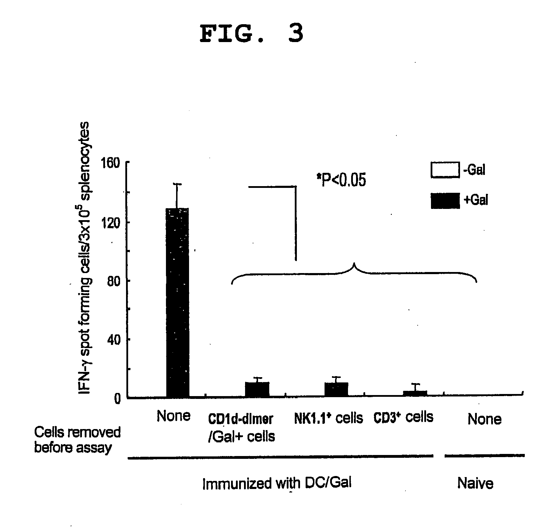

[Figure 3] A graph showing that the IFN-γ secretion is dependent on the presence of NKT cells. To determine whether or not the IFN-γ spots are dependent on NKT cells, NK1.1⁺ cells, CD3⁺ cells, or CD1d-dimer ⁺ cells were removed from splenocytes collected from mice receiving 1×10⁶ cells of hDC/Gal. These cells were cultured as described, and analyzed by ELISPOT assay. The data shown are means for three independent experiments. (^{*}) indicates p<0.05.

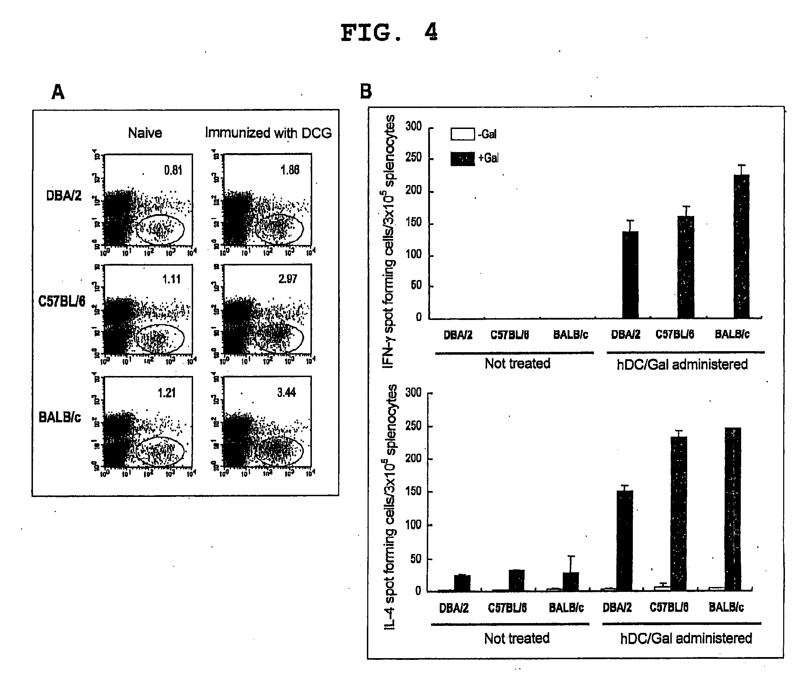

[Figure 4] A drawing showing that human DC/Gal is capable of activating NKT cells of various genetic lines of mice. NKT cell activation after administration of hDC/Gal was evaluated in C57BL/6, DBA/2 and BALB/c mice. Two days after the administration, splenocytes were collected, and NKT cell counts were analyzed by FACS using CD1d-Gal-dimer-PE and CD19-FITC (CD1d-dimer ⁺CD19⁻ cells) (A). Likewise, secretion of IFN-γ and IL-4 was analyzed by ELISPOT assay (B). The data are means for three independent experiments.

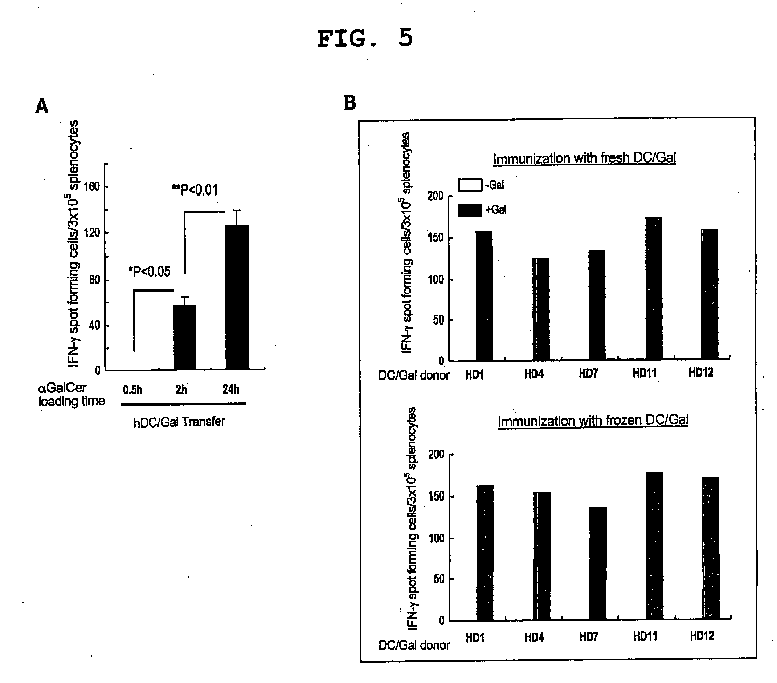

[Figure 5] A drawing showing that the activation of mouse NKT cells is dependent on the αGC loading status on hDC. (A) Effects of hDC incubated along with αGC for various hours were evaluated. After incubation along with αGC for 0.5 hours, 2 hours or 24 hours, hDC was administered to C57BL/6 mice. Two days later, splenocytes were collected, and ELISPOT assay was performed as described above. (B) Fresh hDC/Gal and hDC/Gal freeze-stored in liquid nitrogen for 2 weeks were administered to C57BL/6 mice. Two days later, splenocytes were collected, and IFN-γ ELISPOT assay was performed. The data shown are means for values obtained in three independent experiments. (^{*}) indicates p<0.05. (^{**}) indicates p<0.01.

### Best Mode for Carrying out the Invention

The present invention provides a method of evaluating the antigen presentation potential of human dendritic cells (hereinafter, also referred to as "hDC") (hereinafter, also referred to as "the method of evaluation of the present invention"). Here, "the antigen presentation potential" is used to mean not only the potential for loading a ligand capable of activating NKT cells onto CD1d molecule on dendritic cell surfaces, for example, an α-galactosylceramide (hereinafter, also referred to as "αGC"), but also the potential of ligand-loaded hDC for activating NKT cells.

The test subject hDC is not particularly limited, as far as it is a human-derived dendritic cell capable of activating NKT cells via αGC; it may be any of myeloid dendritic cells (DC1) and lymphoid dendritic cells (DC2), and is preferably DC1. The hDC may be prepared by any method known per se; although it can be separated from the human epidermis, T-cell region of lymphatic tissue, peripheral non-lymphatic tissue, afferent lymph, dermis and the like, it is preferably prepared by, for example, separating monocytes, myelocytes and the like from human peripheral blood and the like by density gradient centrifugation and the like, and culturing them in the presence of GM-CSF and IL-4 for about 7 to about 10 days.

The αGC used to pulse the hDC includes not only α-galactosylceramides, salts thereof, esters thereof and the like, but also all glycolipid derivatives such as CD1d-binding α-C-GalCer, and, as far as they are capable of activating dendritic cells and being antigen-presented to NKT cells, optionally chosen derivatives thereof (for example, synthetic lipids with shortened aliphatic chains, such as OCH, and the like). These can be synthesized by a method known per se. When the hDC is intended for administration to humans, it is desirable that the αGC used be of GMP grade.
Pulsation of hDC with αGC can be performed by an obvious commonly used technique; for example, the pulsation can be performed by culturing the hDC in a medium containing αGC at a concentration of about 100 to about 200 ng/ml (for example, RPMI-1640 medium and the like) for about 12 to about 48 hours. Pulsation with αGC may be performed by adding the αGC to the medium in the process of culturing and maturing the above-described immature hDC in the presence of GM-CSF and IL-4.

The non-human mammal to which αGC-pulsed hDC is administered is not particularly limited; mice, rats, dogs, monkeys and the like are preferable, with greater preference given to mice. In the case of mice, there is no particular limitation on the line; as preferable examples, C57BL/6, DBA/2, BALB/c mice and the like can be mentioned. Although sex and age are also not subject to limitations, it is preferable, in the case of mice, for example, to use ones at about 6 to about 8 weeks of age. Although there is no particular limitation on the rearing environment for the non-human mammal, it is desirable that mice of SPF grade or higher be used.

The hDC may be administered to the recipient non-human mammal just after being pulsed with αGC, and may also be stored under freezing by a method in common use for freeze-storage of dendritic cells (for example, storage in liquid nitrogen), and used by being thawed before use.

The route and dosage for administration of the hDC are not particularly limited, as far as the NKT cells of the recipient non-human mammal can be activated; for example, in the case of mice, it is preferable that about 10⁶ to about 2×10⁶ cells be administered by intravenous administration, intraperitoneal administration and the like.

After administration of hDC, the non-human mammal is reared under ordinary rearing conditions for a time sufficient to activate the NKT cells, after which a sample containing NKT cells is collected from the animal. The sample containing NKT cells is not particularly limited; for example, the sample is derived from a spleen, liver, or peripheral blood, and is preferably derived from a spleen or liver. For example, when a sample derived from a mouse spleen is used, one prepared by resecting the spleen from a mouse about 2 to about 4 days after administration of hDC, and releasing the cells, can be used in the subsequent step for detecting the activation of NKT cells. Alternatively, NKT cells may be sorted in advance using a specific ligand such as αGC.

The presence or absence of activation of NKT cells and the degree thereof can also be measured by any method known per se; for example, the activation of NKT cells can be evaluated with the amount of cytokine produced by the activated NKT cells as an index. As the cytokine produced by the activated NKT cells, IFN-γ, IL-4, GM-CSF, IL-10 and the like can be mentioned. Preferably, the cytokine is IFN-γ or IL-4. Although only one kind of cytokine may be measured, or two kinds or more may be measured, it is desirable that at least one kind be IFN-γ or IL-4.

Cytokine production in NKT cells can be measured using, for example, an antibody against the cytokine. For example, the activation of NKT cells can be evaluated by an immunoassay in common use such as RIA, FIA or EIA using a cell culture supernatant; however, in a preferred mode of embodiment, a method is used wherein a sample containing NKT cells is brought into contact with a solid phase on which an anti-cytokine antibody is immobilized, the solid and liquid are separated, and thereafter the cytokine bound to the solid phase is detected and counted by a sandwich technique using a labeled anti-cytokine antibody. As labeling agents, enzymes, fluorescent substances, luminescent substances, dyes, radioisotopes and the like can be mentioned. A biotinylated anti-cytokine antibody and a (strept)avidin coupled with a labeling agent may be used. An assay system using an enzyme such as alkaline phosphatase as the labeling agent is known under the name ELISPOT for detection of cytokine-producing cells.

Before the sample containing NKT cells is brought into contact with the anti-cytokine antibody, or while the sample is in contact with the anti-cytokine antibody, the NKT cells are re-stimulated with αGC. Although it is desirable that for control, cytokine production be measured also on NKT cells not re-stimulated with αGC, this measurement can be skipped, when using as the index a cytokine whose production without re-stimulation with αGC is known to be under the limit of detection.

For example, in the above-described ELISPOT assay, if the number of spots indicating cytokine-producing cells increases significantly with re-stimulation with αGC, compared with the number obtained without the re-stimulation, the test subject hDC can be assessed as those possessing an antigen presentation potential for NKT cells.
According to the method of evaluation of the present invention, NKT cells are activated with dependency on the dose of hDC, so not only a qualitative evaluation, but also a quantitative evaluation, of hDC is possible. Furthermore, the activation of non-human mammal NKT cells by hDC also correlates with the activation of NKT cells by recipient-derived DC with a good quantifiability, so it is also possible to correct the variation in evaluation due to individual differences in the recipient animal.

The method of evaluation of the present invention enables a determination of the presence or absence of the antigen presentation potential of hDC and the degree thereof without actual administration to a human, so it can be used to sort out hDC to be used in NKT cell immunotherapy. For example, there possibly exists hDC that exhibits only a poor αGC loading rate even when pulsed with αGC under common conditions, or hDC incapable of activating NKT cells even with a good αGC loading rate, so not all the pulsed hDC efficiently presents antigen to NKT cells. Therefore, it is preferable that before performing NKT cell immunotherapy, effective hDC be sorted out in advance. In particular, in performing autotransplantation, if it can be determined in advance whether or not the patient-derived DC possess a sufficient antigen presentation potential to activate NKT cells, it is of paramount significance in determining a therapeutic strategy. That is, if the method of evaluation of the present invention is performed on DC isolated from peripheral blood and the like of a patient who is a subject of application of NKT cell immunotherapy by the same technique as the above, and as a result, the patient's DC is assessed as those possessing a sufficient antigen presentation potential for NKT cells, it can be assessed that NKT cell immunotherapy by autotransplantation can be chosen, or is preferably chosen; if the DC is assessed as those which do not possess a sufficient antigen presentation potential for NKT cells, it can be assessed that allogeneic transplantation or a therapy other than NKT cell immunotherapy should be chosen, or is preferably chosen.

Also, as stated above, the method of evaluation of the present invention is quantitative, so it can be used to evaluate the degree of the potential of hDC for activating NKT cells. For example, when allogeneic transplantation is chosen, hDC with a higher potential for activating NKT cells can be sorted out using the method of evaluation of the present invention. Also, it is possible to determine the number of hDC administered, on the basis of individual hDC potential values. Furthermore, when hDC in long storage under freezing is used by being thawed before use, it can be used to confirm the maintenance of the NKT cell activation potential for the purpose of implementing quality control of the hDC before administration.

The method of evaluation of the present invention can also be used to perform monitoring of the efficacy of a treatment in a patient undergoing NKT cell immunotherapy. For example, by evaluating and comparing the NKT cell activation potentials of patient-derived hDC obtained before and after the start of the immunotherapy, it is possible to determine whether or not the NKT cell immunotherapy is effective on the patient. In this case, an evaluation of hDC can be performed at each time of isolation from the patient, and an evaluation of antigen presentation potential can be performed on hDC isolated before the start of the treatment, and stored under freezing, simultaneously with an evaluation on hDC isolated after the start of the treatment.

As examples of diseases for which NKT cell immunotherapy is indicated, cancers, diabetes (type I diabetes), allergic diseases, rheumatoid arthritis, autoimmune diseases such as collagen disease, bronchial asthma and the like can be mentioned, but these are not limiting, as far as they are diseases in which symptoms can be modified by activation of NKT cells by hDC. As the cancers, all kinds of primary cancers can be mentioned, and all states of cancers, including early cancers and advanced cancers possessing a metastatic or infiltration potential, can be mentioned.

The present invention also provides an agent for human NKT cell immunotherapy, comprising hDC assessed as those possessing an antigen presentation potential by the method of evaluation of the present invention as an active ingredient. Here, "an agent for human NKT cell immunotherapy" means a pharmaceutical capable of activating NKT cells in the body of the human subject of administration as a result of administration of the agent. The agent for human NKT cell immunotherapy of the present invention (hereinafter, also referred to as "the agent of the present invention") is useful in, for example, prevention/treatment of the above-described various diseases in humans.

The agent of the present invention can be produced as an oral/parenteral preparation by blending an effective amount of the above-described αGC-pulsed hDC with a pharmaceutically acceptable carrier and the like by a conventional method. The hDC may be one that is newly isolated from a human from whom hDC assessed as those possessing an antigen presentation potential by the method of evaluation of the present invention is derived, cultured in the same manner as the above, and pulsed with αGC, or αGC-pulsed hDC may be partially used in the above-described method of evaluation, and the remaining may be stored under freezing and used by being thawed before use.

The agent of the present invention is usually produced as a parenteral preparation such as an injection, suspension, or drip infusion. As examples of pharmaceutically acceptable carriers that can be contained in the parenteral preparation, aqueous liquids for injection, such as physiological saline and isotonic solutions containing glucose and other auxiliary drugs (for example, D-sorbitol, D-mannitol, sodium chloride and the like) can be mentioned. The agent of the present invention may be formulated with, for example, a buffering agent (for example, phosphate buffer solution, sodium acetate buffer solution), a soothing agent (for example, benzalkonium chloride, procaine hydrochloride and the like), a stabilizer (for example, human serum albumin, polyethylene glycol and the like), a preservative, an anti-oxidant and the like.

When the agent of the present invention is prepared as an aqueous suspension, αGC-pulsed hDC is suspended in the above-described aqueous liquid to obtain a cell density of about 5×10⁶ to about 1×10⁷ cells/ml.

Because the preparation thus obtained is safe and less toxic, it can be safely administered to humans. Although it is preferable that the subject of administration be the patient from which the hDC is derived (i.e., autotransplantation), the subject of administration is not limited thereto, as far as it is a human expected to be compatible with the hDC administered. The method of administration is not particularly limited; the agent of the present invention can be administered orally or parenterally, preferably by injection or drip infusion; intravenous administration, subcutaneous administration, intradermal administration, intramuscular administration, intraperitoneal administration, direct administration to the affected site and the like can be mentioned. The dose of the agent of the present invention varies depending on the subject of administration, target organ, symptoms, method of administration and the like; usually in an adult patient (assuming a 60 kg body weight), in the case of parenteral administration, for example, it is convenient to administer the preparation usually in an amount of about 6×10⁵ to about 1×10⁷ cells, based on the amount of hDC per dose, at intervals of about 1 to about 2 weeks, about 4 to about 8 times.

The present invention also provides a method of screening for a substance capable of activating human NKT cells, or capable of regulating (inhibiting and enhancing) the activation by αGC. The method comprises the following steps.
(a) A step for administering hDC pulsed with a test substance to a non-human mammal;
(b) a step for collecting from the non-human mammal a sample containing NKT cells;
(c) a step for detecting the activation of NKT cells present in the sample.

The test substance used in screening in the step (a) of the method of screening of the present invention can be an optionally chosen publicly known compound, a novel compound, or a library of such compounds. As examples of the compounds, proteins (oligopeptides, polypepetides and the like), sugar chains (bacterial polysaccharides and the like), glycolipids (blood group substances and the like), glycoproteins, lipids, nucleic acids (DNA, RNA and the like), simple chemical substances (low-molecular chemical substances and the like) and the like can be mentioned. The test substances used for the screening can be simultaneously pulsed singly or in combination of a plurality thereof (2 to 1000 test substances and the like). For example, in the compound library screening step, by reducing the number of candidate test substances to be combined, a final candidate test substance can be identified. When a substance capable of regulating the activation by αGC is screened for, αGC is pulsed to hDC along with a test substance.

The hDC used in the step (a) is desirably one that has previously been confirmed as being capable of activating NKT cells when pulsed with αGC. However, even in that case, to accurately compare the performances, i.e., the potentials for activating NKT cells, of the test substance and αGC as ligands, and also to confirm the maintenance of the antigen presentation potential of the hDC, it is preferable that the steps (a) to (c) be performed in the same manner using αGC in place of the test substance.

In the method of screening of the present invention, the steps (a) to (c) can be performed in the same ways as with the above-described method of evaluation of the present invention. When the activation of NKT cells present in the sample containing NKT cells is performed using an anti-cytokine antibody with a cytokine produced by activated NKT cells as an index in the step (c), the NKT cells are re-stimulated with a single or a plurality of pulsed test substances before being brought into contact with the anti-cytokine antibody, or while in contact with the anti-cytokine antibody. It is desirable that for control, cytokine production be also measured for NKT cells not stimulated with the test substance.

For example, in the above-described ELISPOT assay, if the number of spots indicating cytokine-producing cells increases significantly with re-stimulation with the test substance, compared with the number obtained without the re-stimulation, the test substance can be assessed as that is capable of acting as a ligand that activates NKT cells. By comparing with the number obtained with pulsation with the positive control αGC, the degree of the potential of the test substance for activating NKT cells can be evaluated quantitatively.
Meanwhile, if a significant change occurs with pulsation with αGC and the test substance, compared with pulsation with αGC alone, the test substance can be assessed as that is capable of regulating the activation of NKT cells by αGC. It can be determined whether the test substance is loaded onto CD1d competitively with αGC, or alters the αGC loading rate or the potential of loaded αGC for activating NKT cells, on the basis of whether or not the hDC is capable of activating NKT cells when pulsed with the test substance alone.

A ligand compound capable of activating NKT cells, or a compound capable of enhancing the potential of αGC for activating the cells, obtained by the above-described method of screening, in place of αGC, or along therewith, can be used in the method of evaluation of the present invention. hDC pulsed with the compound, or with the compound and αGC, can be formulated as an active ingredient in the above-described agent for human NKT cell immunotherapy.

### Examples

The present invention is hereinafter described in more specifically with reference to the following Examples, which, however, are for illustrative purposes only, and never limit the scope of the present invention.

### (Collection and preparation of samples)

Peripheral blood mononuclear cells (PBMC) were separated from venous blood drawn from a healthy blood donor by Ficoll-Hypaque (GE Healthcare Biosciences, Uppsala, Sweden) density gradient centrifugation. The PBMCs were washed with PBS three times, and stored in a liquid nitrogen tank until use.
Informed consent in writing was obtained from the patient in compliance with the Helsinki Declaration. All the research was approved by RIKEN's Institutional Review Board.

### (Mice)

Sterile female C57BL/6, DBA/2 and BALB/c mice at 6 to 8 weeks of age were purchased from CLEA Japan (Tokyo). These mice and Jα18^{-/-} mice were reared and maintained under SPF conditions, and research was conducted in accordance with the institutes' guidelines.

### (Reagents)

αGC was synthesized at RIKEN. αGC and a vehicle (0.4% DMSO) were diluted in PBS. Human recombinant (r) GM-CSF and IL-4 were purchased from R&D systems (Minneapolis, MN). An anti-mouse-CD19 monoclonal antibody (1D3), anti-TCRβ monoclonal antibody (H57-597) and mouse IgG1 (A85-1) were purchased from BD PharMingen (San Diego, CA). In the flow cytometry of NKT cells, the recombinant soluble dimer mouse CD1d:Ig (BD PharMingen)-PE and an FITC-labeled anti-mouse CD19 antibody were used. In the analysis, the FACS Calibur^{™} apparatus and CELLQuest^{™} (BD Biosciences) were used.

### (Generation of human and mouse DC)

Using magnetic beads (Miltenyi Biotec Inc.Auburn, CA), CD14⁺ monocytes were purified from the PBMCs, and cultured in the presence of 500 U/ml human IL-4 and 100 ng/ml human GM-CSF for 7 days. The αGC (100 ng/ml) or an equivalent quantity of the vehicle was added to the hDC on day 6, and the cells were further cultured for 24 hours. Mouse bone marrow-derived DC was proliferated from a bone marrow precursor in an RPMI-1640 containing 5% FCS and mouse GM-CSF, as reported previously (J. Exp. Med. 176, 1693-1702, 1992). On day 6, αGC (100 ng/ml) was added to immature DC; in many experiments, the cells were further cultured for 2 days. The αGC-pulsed DC (DC/Gal) was collected on day 8.

### (Single-cell ELISPOT assay for quantifying the amount of cytokines produced in NKT cells in spleens of immunized mice)

A 96-well titer plate (Millipore, Bedford, MA) was coated with an anti-mouse IFN-γ monoclonal antibody or anti-mouse IL-4 monoclonal antibody (both 10 µg/ml, BD PharMingen). After immunization with the human DC/Gal for 2 days, splenocytes (3×10⁵ cells /well) were cultured along with either the vehicle or αGC (100 ng/ml) for 16 hours. After the cultivation, the plate was washed, and incubated with biotinylated anti-mouse IFN-γ monoclonal antibody or anti-mouse IL-4 monoclonal antibody (both 1 µg/ml, BD PharMingen). IFN-γ or IL-4 spot-forming cells (SFC) were counted under a microscope.

### (Statistical analysis)

Data differences were analyzed using Mann-Whitney U-test. P<0.05 is considered to indicate a statistically significant difference.

### Example 1 Activation of NKT cells with hDC in vitro and in vivo

To stimulate C57BL/6-derived liver mononuclear cells, αGC-loaded human or mouse DC was used (Figure 1). In vitro, the mouse DC stimulated NKT cells with dependence on αGC, and IFN-γ was produced, whereas the human DC did not efficiently stimulate NKT cells (Figure 1A). Next, two days after administration of αGC-pulsed mouse DC (mDC/Gal) or human DC (hDC/Gal), NKT cell reactions in the immunized mice were tested (Figure 1B). As a result, in the mice receiving hDC/Gal, more than 100 αGC-specific IFN-γ spots were detected. From this result, it was shown that this technique is useful in evaluating the DC function on NKT cell stimulation. It was thought that when human DC was administered to mice, nonspecific responses would be produced; however, an unexpected result was obtained in which the human DC could be used for the evaluation as shown in Figure 1.

### Example 2 Dose dependence of activation of mouse NKT cells by administration of hDC/Gal

Two days after αGC-pulsed hDC was administered to C57BL/6 mice at stepwise doses, production of IFN-γ and IL-4 by NKT cells was examined (Figure 2A). When non-pulsed hDC was administered to mice, no IFN-γ spots were detected. In the mice receiving hDC/Gal, the number of IFN-γ production spots increased in a correlation with the number of hDC/Gal administered; 1×10⁶ cells was determined to be the appropriate dose. When 1×10⁶ cells of hDC/Gal were administered to Jα18^{-/-} mice, which are NKT cell-deficient mice, no spots of IFN-γ or IL-4 production were observed (Figure 2B). This showed that the observed cytokine production is dependent on the interaction of ligand and NKT cells.

### Example 3 Confirmation of NKT cell dependency in in vitro functional assay

An investigation was made to determine whether or not the IFN-γ spots observed in the ELISPOT assay were dependent on the presence of NKT cells and ligand (Figure 3). It was found that when NK1.1⁺ cells, CD3⁺ cells, or CD1d-dimer ⁺ cells derived from splenocytes of mice receiving 1×10⁶ cells of hDC/Gal were removed before performing the ELISPOT assay, almost all spots disappeared. This showed that IFN-γ-producing cells were dependent on at least NK1.1⁺CD3⁺CD1d-dimer⁺ cells.

### Example 4 Confirmation of non-dependence on mouse line of activation of NKT cells by hDC/Gal

To determine whether or not the activation of NKT cells is dependent on mouse line, NKT cell reactions to hDC/Gal were tested in various lines of mice. Two days after administration of hDC/Gal, splenocytes derived from C57BL/6, DBA/2 and BALB/c mice were collected, and FACS analysis was performed. It was revealed that in all mouse lines, the NKT cell count had increased 2 folds or more (Figure 4A). It was also found that in all the mice of the tested lines, cells producing IFN-γ and IL-4 had increased similarly (Figure 4B). From these results, it was shown that the success of the experiments was not attributable solely to the specific genetic background of the mice, that is, C57BL/6, but many lines of wild-type mice could be used in evaluating the efficacy of hDC using this method.

### Example 5

### (a) Investigation of αGC pulse conditions

After hDC was cultured along with αGC for 0.5 hours, 2 hours or 24 hours, the potential of the hDC for activating mouse NKT cells in vivo was tested. Although the IFN-γ-producing NKT cells in mice receiving hDC pulsed with αGC for 0.5 hours exhibited nearly zero activity, mice receiving hDC pulsed with αGC for 2 hours exhibited an activity roughly half the level in mice receiving hDC pulsed with αGC for 24 hours (Figure 5A).

### (b) Stability test of hDC/Gal

Furthermore, a stability test was conducted to determine whether or not there was a difference in the activation of NKT cells in mice receiving a fresh hDC/Gal culture, compared with mice receiving hDC/Gal stored in liquid nitrogen for 2 weeks. From the results shown in Figure 5B, both the fresh hDC and previously frozen hDC were shown to exhibit similar degrees of NKT cell reactions. This demonstrates that hDC once loaded with αGC is highly stable, and can be used for a long time when stored under freezing.

### Industrial Applicability

Using the method of the present invention, it is possible to sort out human dendritic cells to be used in human NKT cell immunotherapy, and it is also possible to evaluate the degree of the antigen presentation potential. The method of the present invention can also be used for efficacy monitoring in patients undergoing human NKT cell immunotherapy. Furthermore, the method of the present invention is also useful as a method of screening for a novel ligand. The agent of the present invention is useful in efficiently degenerating cancers.

This application is based on a patent application No. 2007-234734 filed in Japan (filing date: September 10, 2007), the contents of which are incorporated in full herein by this reference.

## Claims

1. A method of evaluating the antigen presentation potential of human dendritic cells, comprising the following steps:
(a) a step for administering α-galactosylceramide-pulsed human dendritic cells to a non-human mammal;
(b) a step for collecting a sample containing NKT cells from the non-human mammal;
(c) a step for detecting the activation of NKT cells present in the sample.

2. The method according to claim 1, wherein the non-human mammal is a mouse.

3. The method according to claim 1 or 2, wherein the sample containing NKT cells is derived from a spleen, liver or peripheral blood.

4. The method according to any one of claims 1 to 3, wherein the activation of NKT cells is evaluated with one or more kinds of cytokines produced by the NKT cells as an index.

5. The method according to claim 4, wherein the cytokines include interferon-γ and/or interleukin-4.

6. The method according to any one of claims 1 to 5, wherein the method is for sorting out human dendritic cells to be used in human NKT cell immunotherapy.

7. The method according to claim 6, wherein the human dendritic cells are derived from a patient with a disease for which a therapeutic effect can be obtained by NKT cell immunotherapy.

8. The method according to claim 7, wherein the disease is a cancer.

9. An agent for human NKT cell immunotherapy, comprising human dendritic cells that have been assessed by the method according to claim 6 or 7 as those possessing an antigen presentation potential for NKT cells.

10. The agent according to claim 9, wherein the agent is for treatment of a cancer.

11. A method of screening for a ligand capable of activating human NKT cells, comprising the following steps:
(a) a step for administering human dendritic cells pulsed with a test substance to a non-human mammal;
(b) a step for collecting a sample containing NKT cells from the non-human mammal;
(c) a step for detecting the activation of NKT cells present in the sample.

12. The method according to claim 10, further comprising performing the foregoing steps (a) to (c) using an α-galactosylceramide in place of the test substance, and comparing the degree of activation of NKT cells between a case where the cells are pulsed with the test substance and a case where the cells are pulsed with an α-galactosylceramide.

13. The method according to claim 11 or 12, wherein the non-human mammal is a mouse.
